# EUROPÄISCHE PATENTSCHRIFT

(11) **EP 1 236 718 B1**
(45) Veröffentlichungstag und Bekanntmachung des Hinweises auf die Patenterteilung: **11.08.2004**
(21) Anmeldenummer: 02002071.5
(22) Anmeldetag: 11.02.2002
(51) Int. Cl.: C07D 215/56

(54) **Verbessertes Verfahren zur Herstellung von Fluorchinoloncarbonsäuren**
Improved process for the preparation of fluoro-quinolonecarboxylic acids
Procédure améliorée pour la préparation des acids carboxiliques de fluoroquinolone

(30) Priorität: 23.02.2001 DE 10108750
(43) Veröffentlichungstag der Anmeldung: 04.09.2002
(73) Patentinhaber: Bayer Chemicals AG, 51368 Leverkusen (DE)
(72) Erfinder: Lui, Norbert, Dr., 51061 Köln (DE); Panskus, Hans, 51373 Leverkusen (DE)

(56) Entgegenhaltungen:
- EP-A- 0 169 993
- EP-A- 0 284 935
- EP-A- 0 653 425
- GB-A- 2 146 638
- HRADIL, PAVEL ET AL.: "Cyclization of phenacyl 2-((2,2-di(ethoxycarbonyl)-vinyl)amino)ben zoate" COLLECT. CZECH. CHEM. COMMUN., Bd. 63, Nr. 4, 1998, Seiten 520-524, XP001069366
- FUJITA, M. ET AL.: "Pyrroloquinolones and pyrazoloquinolones as potential antibacterial agents. Synthesis and antibacterial activity" EUR. J. MED. CHEM. CHIM. THER. (EN), Bd. 31, Nr. 12, 1996, Seiten 981-988, XP004071793
- HOUBEN-WEYL: "Methoden der Organischen Chemie, Band VIII, Sauerstoffverbindungen III" 1952 , GEORG THIEME VERLAG , STUTTGART XP002197597 Kapitel 4.III, Herstellung von Carbonsäuren durch Umwandlung von Carbonsäurederivaten, Seiten 418-421 * Seite 419, Zeile 13,14 *

## Beschreibung

Die vorliegende Erfindung betrifft ein vorteilhaftes Verfahren zur Herstellung von Fluorchinoloncarbonsäuren durch saure Verseifung der entsprechenden Fluorchinoloncarbonsäureester.

Fluorchinoloncarbonsäuren sind wichtige Zwischenprodukte zur Herstellung von bekannten pharmazeutisch wirksamen Verbindungen aus der Klasse der Chinolone.

Es ist bekannt (siehe EP-A 169 993), dass man Cyclopropyl-6-fluor-1,4-dihydro-4-oxo-chinolincarbonsäureester unter sauren oder basischen Bedingungen zu den entsprechenden Chinoloncarbonsäuren verseifen kann (loc. cit. Seite 10, Zeilen 4 bis 7). Konkreter beschrieben ist nur die Verseifung von 94 g 1-Cyclopropyl-6,7,8-trifluor-1,4-dihydroxy-4-oxo-3-chinolincarbonsäureethylester unter Zusatz von Wasser, Eisessig und 70 ml = 128,8 g konzentrierter Schwefelsäure (das entspricht pro Mol des Fluorchinoloncarbonsäureesters ca. 420 g Schwefelsäure), wobei man das Reaktionsgemisch für 1,5 Stunden auf Rückfluss erhitzt, anschließend die vorliegende Suspension auf Eis gießt und den dann vorliegenden Niederschlag absaugt, wäscht und trocknet (loc. cit. Seite 28, letzter Absatz). Die Ausbeute beträgt dabei 96 % der Theorie.

Nachteilig bei diesem Verfahren ist die große Menge Schwefelsäure die benötigt wird, die großen Abwassermengen und die sich damit ergebenden Entsorgungsprobleme, die sich aus dem Einsatz von großen Mengen Schwefelsäure (diese fällt dann als Dünnsäure an) und Eis ergeben und die Notwendigkeit, das isolierte Produkt mehrmals zu waschen, um anhaftende Schwefelsäurereste zu entfernen.

Es wurde nun ein Verfahren zur Herstellung von Fluorchinoloncarbonsäuren durch Verseifung der entsprechenden C₁-C₄-Alkylester unter Zusatz von Wasser, Essigsäure und Schwefelsäure gefunden, das dadurch gekennzeichnet ist, dass man bezogen auf 1 Mol Fluorchinoloncarbonsäure-C₁-C₄-alkylester weniger als 30 g Schwefelsäure einsetzt, das Reaktionsgemisch für 0,5 bis 8 Stunden auf Rückfluss erhitzt, dann ein Gemisch aus Essigsäure, C₁-C₄-Alkylacetat, C₁-C₄-Alkylalkohol und gegebenenfalls Wasser abdestilliert und abschließend die hergestellte Fluorchinoloncarbonsäure isoliert.

Als einzusetzende Fluorchinoloncarbonsäure-C₁-C₄-alkylester kommen beispielsweise solche der Formel (I) in Frage 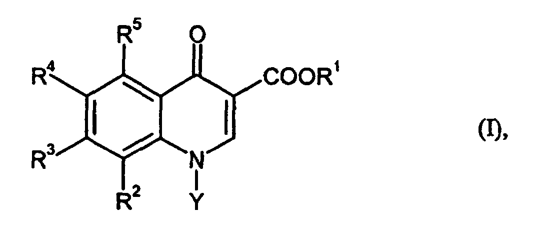 in der
- R¹: für C₁-C₄-Alkyl steht,
- R²: für Wasserstoff, C₁-C₄-Alkyl, C₁-C₄-Alkoxy, Halogen, Nitro oder Cyano steht,
- R³ und R⁴: je für ein Halogen stehen,
- R⁵: für Wasserstoff, C₁-C₄-Alkyl, Halogen oder Nitro steht, und
- Y: für C₁-C₆-Alkyl, Cyclopropyl oder Phenyl steht, die gegebenenfalls mit Halogen substituiert sein können,
wobei R² und Y gemeinsam auch für eine mit einem C-Atom an das Stickstoffatom gebundene -CH₂-CH₂-O- oder -CH(CH₃)-CH₂-O-Brücke stehen können und wobei wenigstens einer der Reste R² bis R⁵ für Fluor steht.

Aus den beispielsweise einzusetzenden Fluorchinoloncarbonsäure-C₁-C₄-alkylestern der Formel (I) kann man die entsprechenden Fluorchinoloncarbonsäuren der Formel (II) erhalten 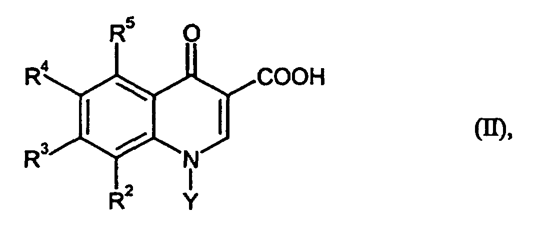 in der die Reste R² bis R⁵ und Y die bei Formel (I) angegebene Bedeutung haben.

Vorzugsweise stehen in den Formeln (I) und (II)
- R²: für Wasserstoff, Methyl, Methoxy, Fluor, Chlor, Nitro oder Cyano,
- R³: für Fluor oder Chlor,
- R⁴: für Fluor,
- R⁵: für Wasserstoff, Methyl, Fluor, Chlor oder Nitro und
- Y: für Methyl, Ethyl, Isopropyl, Cyclopropyl, Fluorcyclopropyl, 4-Fluorphenyl oder 2,4-Difluorphenyl.

Vorzugsweise steht in Formel (I) R¹ für Methyl oder Ethyl.

In das erfindungsgemäße Verfahren kann man Essigsäure und Schwefelsäure in wasserhaltiger oder wasserfreier Form einsetzen. Die beschriebenen Mengenangaben beziehen sich auf 100 %ige Essigsäure und 100 %ige Schwefelsäure. Wenn man Wasser enthaltende Essigsäure und/oder Wasser enthaltende Schwefelsäure einsetzt, muss man entsprechend deren Wassergehalt weniger Wasser einsetzen. Essigsäure wird vorzugsweise in Form von Eisessig, Schwefelsäure vorzugsweise in Form von 96 bis 100 %iger Schwefelsäure eingesetzt.

Bezogen auf 1 Mol Fluorchinoloncarbonsäure-C₁-C₄-alkylester kann man in das erfindungsgemäße Verfahren beispielsweise 20 bis 250 ml Wasser, 200 bis 2000 ml Essigsäure und 2 bis 25 g Schwefelsäure einsetzen. Bevorzugt betragen diese Mengen 100 bis 200 ml Wasser, 300 bis 1000 ml Essigsäure und 3 bis 15 g Schwefelsäure.

Die Zugabe von Wasser, Essigsäure und Schwefelsäure erfolgt vorzugsweise so, dass man den Fluorchinoloncarbonsäure-C₁-C₄-alkylester, das Wasser und die Essigsäure vorlegt und anschließend die Schwefelsäure zufügt.

Vorzugsweise wird das Reaktionsgemisch für 1 bis 5 Stunden auf Rückfluss erhitzt.

Nach Beendigung des Erhitzens auf Rückfluss werden aus dem Reaktionsgemisch Essigsäure, C₁-C₄-Alkylacetat, C₁-C₄-Alkylalkohol und Wasser abdestilliert. Die Destillation kann man beispielsweise so lange betreiben, bis sich eine Sumpftemperatur im Bereich von 107 bis 113°C ergibt. Vorzugsweise betreibt man die Destillation so lange, bis sich eine Sumpftemperatur im Bereich von 108 bis 110°C ergibt. Diese Temperaturen beziehen sich auf Normaldruck. Arbeitet man bei anderen Drucken so sind diese Temperaturen entsprechend niedriger oder höher anzusetzen.

Die bei der Destillation abdestillierenden 3 bis 4 Komponenten destillieren i.a. in Form von Azeotropen, deren Zusammensetzung sich während der Destillation ändern kann.

Das Erhitzen des Reaktionsgemisches auf Rückfluss und die anschließende Destillation können bei vermindertem Druck, atmosphärischem Druck oder erhöhtem Druck durchgeführt werden. Beispielsweise sind Drücke im Bereich von 0,5 bis 3 bar möglich. Vorzugsweise arbeitet man in beiden Verfahrensschritten bei atmosphärischem Druck.

Die Isolierung der hergestellten Fluorchinoloncarbonsäure aus dem nach der Destillation vorliegenden Gemisch kann beispielsweise so erfolgen, dass man dieses Gemisch mit Wasser verdünnt und den dann vorliegenden Niederschlag absaugt, mit Wasser wäscht und trocknet. Es ist vorteilhaft, in diesem Falle das isolierte Produkt mehrmals zu waschen, um es hinreichend frei und weitgehend ohne anhaftende Schwefelsäure zu erhalten.

Bevorzugt führt man die Isolierung der hergestellten Fluorchinoloncarbonsäure durch, indem man in dem nach der Destillation vorliegenden Gemisch durch Zugabe einer Base einen pH-Wert im Bereich von 2 bis 5, vorzugsweise 3 bis 4 einstellt. Das kann beispielsweise durch Zusatz einer entsprechenden Menge Natronlauge oder Natriumacetat erreicht werden. Vorzugsweise verwendet man hier eine 1 bis 20 gew.-%ige wässrige Natriumacetat-Lösung. Den für die Isolierung einer bestimmten Fluorchinoloncarbonsäure optimalen pH-Wert kann man durch eine einfache Titration ermitteln. Man nimmt dann den sich aus der Titration ergebenden pH-Wert, der einerseits möglichst hoch liegt, andererseits aber noch nicht zur Ausfällung von Salzen der jeweiligen Fluorchinoloncarbonsäure führt. Man kann nach der Einstellung des pH-Wertes das Gemisch beispielsweise auf 0 bis 35°C abkühlen und den dann vorliegenden Niederschlag abfiltrieren, mit Wasser waschen und trocknen. Die Trocknung wird vorzugsweise bei erhöhter Temperatur und vermindertem Druck durchgeführt. Man erhält dann in der Regel schon mit einmaligem Waschen ein hinreichend reines Produkt.

Mit dem erfindungsgemäßen Verfahren erhält man Fluorchinoloncarbonsäuren im allgemeinen in Ausbeuten von 98 % der Theorie und höher.

Auf die erfindungsgemäße Art und Weise können insbesondere folgende Fluorchinoloncarbonsäuren in vorteilhafter Weise erhalten werden:
1-Cyclopropyl-6,7,8-trifluor-1,4-dihydro-4-oxo-3-chinolin-carbonsäure,
1-Cyclopropyl-6,7-difluor-1,4-dihydro-4-oxo-3-chinolin-carbonsäure,
1-Cyclopropyl-6,7-difluor-8-cyano-1,4-dihydro-4-oxo-3-chinolin-carbonsäure,
1-(2-Fluor)cyclopropyl-6,7-difluor-1,4-dihydro-4-oxo-3-chinolin-carbonsäure,
1-Cyclopropyl-8-chlor-6,7-difluor-1,4-dihydro-4-oxo-3-chinolin-carbonsäure und
1-Ethyl-6,7,8-trifluor-1,4-dihydro-4-oxo-3-chinolin-carbonsäure.

Das erfindungsgemäße Verfahren hat den Vorteil, dass bei ihm gegenüber dem Stand der Technik sehr viel weniger Schwefelsäure (nur ca. 1 bis 10 % der bisher angewendeten Menge) eingesetzt wird und deshalb die anfallende Menge Dünnsäure wesentlich geringer ist. Wendet man auch die vorteilhafte, pH-kontrollierte Methode zur Isolierung der hergestellten Fluorchinoloncarbonsäuren an, so kann man Waschvorgänge vermeiden und weitere Verminderungen der Abwassermenge realisieren. Schließlich ist die Ausbeute an gewünschtem Produkt höher als bisher.

Die erfindungsgemäß sich ergebenden Vorteile sind sehr überraschend, denn wenn man gegenüber dem Stand der Technik nur die Schwefelsäuremenge reduziert, die Destillation jedoch nicht durchführt oder die Schwefelsäuremenge reduziert und nur kurz auf Rückfluss erhitzt, dann läuft die Verseifung nicht mehr nahezu quantitativ, sondern nur noch unvollständig ab, was zu Ausbeuteverminderungen und Produktverunreinigungen führt (siehe Vergleichsbeispiele).

### Beispiele

### Beispiel 1

300 g 1-Cyclopropyl-6,7,8-trifluor-1,4-dihydro-4-oxo-3-chinolin-carbonsäureethylester, 106,8 g Wasser und 426 g Essigsäure wurden vorgelegt und 3,8 g Schwefelsäure zugesetzt. Die Mischung wurde 3 Stunden auf Rückfluss erhitzt. Anschließend wurden bis zum Erreichen einer Sumpftemperatur von 109°C 310 ml Destillat abdestilliert. Dann wurde das Gemisch auf 80°C abgekühlt und 157,5 g 4,8 gew.-%ige Natriumacetatlösung zugetropft. Der pH-Wert lag dann im Bereich 3 bis 4. Das Gemisch wurde anschließend auf 20°C abgekühlt und der Feststoff abgesaugt. Der Feststoff wurde mit 200 ml Wasser gewaschen und im Vakuum bei 50°C getrocknet. Es wurden 270,3 g 1-Cyclopropyl-6,7,8-trifluor-1,4-dihydro-4-oxo-3-chinolin-carbonsäure isoliert, was einer Ausbeute von 99 % der Theorie entspricht.

### Beispiel 2

1500 g 1-Cyclopropyl-6,7-difluor-1,4-dihydro-4-oxo-3-chinolin-carbonsäureethylester, 128,4 g Wasser und 4500 g Essigsäure wurden vorgelegt und 53 g Schwefelsäure zugesetzt. Die Mischung wurde 4 Stunden auf Rückfluss erhitzt. Anschließend wurden bis zum Erreichen einer Sumpftemperatur von 109°C 2020 ml Destillat abdestilliert. Die Suspension wurde auf 80°C abgekühlt und 2204 g 4 Gew.-%ige wässrige Natriumacetatlösung zugetropft. Der pH-Wert lag dann im Bereich 3 bis 4. Die Reaktionsmischung wurde anschließend auf 20°C abgekühlt und der Feststoff abgesaugt. Der Feststoff wurde mit 2000 ml Wasser gewaschen und im Vakuum bei 50°C getrocknet. Es wurden 1329 g 1-Cyclopropyl-6,7-difluor-1,4-dihydro-4-oxo-3-chinolin-carbonsäure isoliert, was einer Ausbeute von 98 % entspricht.

### Vergleichsbeispiel 1

300 g 1-Cyclopropyl-6,7,8-trifluor-1,4-dihydro-4-oxo-3-chinolin-carbonsäureethylester, 106,8 g Wasser und 426 g Essigsäure wurden vorgelegt und 3,8 g Schwefelsäure zugesetzt. Die Mischung wurde auf Rückfluss erhitzt und sofort 310 ml Destillat abdestilliert. Die Suspension wurde auf 80°C abgekühlt und 157,5 g 4,8 %ige wässrige Natriumacetatlösung zugetropft. Die Reaktionsmischung wurde anschließend auf 20°C abgekühlt und der Feststoff abgesaugt. Der Feststoff wurde mit 200 ml Wasser gewaschen und im Vakuum bei 50°C getrocknet. Der Feststoff bestand aus einer Mischung von 1-Cyclopropyl-6,7,8-trifluor-1,4-dihydro-4-oxo-3-chinolin-carbonsäureethylester (8 %) und 1-Cyclopropyl-6,7,8-trifluor-1,4-dihydro-4-oxo-3-chinolin-carbonsäure (92 %).

Dieses Vergleichsbeispiel zeigt, der Einsatz einer kleinen Menge Schwefelsäure und nur ein kurzes Erhitzen auf Rückfluss erbringt schlechtere Ergebnisse als der Stand der Technik.

### Vergleichsbeispiel 2

300 g 1-Cyclopropyl-6,7,8-trifluor-1,4-dihydro-4-oxo-3-chinolin-carbonsäureethylester, 106,8 g Wasser und 426 g Essigsäure wurden vorgelegt und 3,8 g Schwefelsäure zugesetzt. Die Mischung wurde 1,5 Stunden auf Rückfluss erhitzt, dann auf 80°C abgekühlt und 157,5 g 4,8 %ige wässrige Natriumacetatlösung zugetropft. Die Reaktionsmischung wurde anschließend auf 20°C abgekühlt und der Feststoff abgesaugt. Der Feststoff wurde mit 200 ml Wasser gewaschen und im Vakuum bei 50°C getrocknet. Der Feststoff bestand aus einer Mischung von 1-Cyclopropyl-6,7,8-trifluor-1,4-dihydro-4-oxo-3-chinolin-carbonsäureethylester (11 %) und 1-Cyclopropyl-6,7,8-trifluor-1,4-dihydro-4-oxo-3-chinolin-carbonsäure (89 %).

Dieses Vergleichsbeispiel zeigt, der Einsatz einer kleinen Menge Schwefelsäure ohne die erfindungsgemäß durchzuführende Destillation erbringt schlechtere Ergebnisse als der Stand der Technik.

## Patentansprüche

1. Verfahren zur Herstellung von Fluorchinoloncarbonsäuren durch Verseifung der entsprechenden C₁-C₄-Alkylester unter Zusatz von Wasser, Essigsäure und Schwefelsäure, **dadurch gekennzeichnet, dass** man bezogen auf 1 Mol Fluorchinoloncarbonsäure-C₁-C₄-alkylester weniger als 30 g Schwefelsäure einsetzt, das Reaktionsgemisch für 0,5 bis 8 Stunden auf Rückfluss erhitzt, dann ein Gemisch aus Essigsäure, C₁-C₄-Alkylacetat, C₁-C₄-Alkylalkohol und gegebenenfalls Wasser abdestilliert und abschließend die hergestellte Fluorchinoloncarbonsäure isoliert.

2. Verfahren nach Anspruch 1, **dadurch gekennzeichnet, dass** man Fluorchinoloncarbonsäure-C₁-C₄-alkylester der Formel 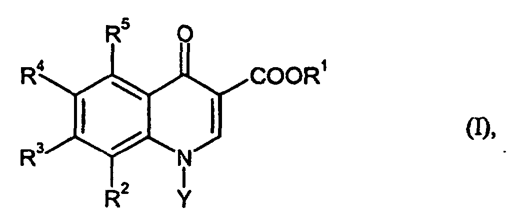 in der
R¹ für C₁-C₄-Alkyl steht,
R² für Wasserstoff, C₁-C₄-Alkyl, C₁-C₄-Alkoxy, Halogen, Nitro oder Cyano steht,
R³ und R⁴ je für ein Halogen stehen,
R⁵ für Wasserstoff, C₁-C₄-Alkyl, Halogen oder Nitro steht, und
Y für C₁-C₆-alkyl, Cyclopropyl oder Phenyl steht, die gegebenenfalls mit Halogen substituiert sein können,
wobei R² und Y gemeinsam auch für eine mit einem C-Atom an das Stickstoffatom gebundene -CH₂-CH₂-O- oder -CH(CH₃)-CH₂-O-Brücke stehen können und
wobei wenigstens einer der Reste R² bis R⁵ für Fluor steht,
einsetzt und Fluorchinoloncarbonsäuren der Formel 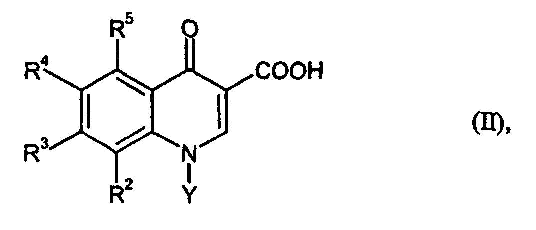 in der die Reste R² bis R⁵ und Y die bei Formel (I) angegebene Bedeutung haben, herstellt.

3. Verfahren nach Anspruch 2, **dadurch gekennzeichnet, dass** in den Formeln (I) und (II)
R² für Wasserstoff, Methyl, Methoxy, Fluor, Chlor, Nitro oder Cyano,
R³ für Fluor oder Chlor,
R⁴ für Fluor,
R⁵ für Wasserstoff, Methyl, Fluor, Chlor oder Nitro und
Y für Methyl, Ethyl, Isopropyl, Cyclopropyl, Fluorcyclopropyl, 4-Fluorphenyl oder 2,4-Difluorphenyl
und in Formel (I) R¹ für Methyl oder Ethyl stehen.

4. Verfahren nach Ansprüchen 1 bis 3, **dadurch gekennzeichnet, dass** man
1-Cyclopropyl-6,7,8-trifluor-1,4-dihydro-4-oxo-3-chinolin-carbonsäure,
1-Cyclopropyl-6,7-difluor-1,4-dihydro-4-oxo-3-chinolin-carbonsäure,
1-Cyclopropyl-6,7-difluor-8-cyano-1,4-dihydro-4-oxo-3-chinolin-carbonsäure,
1-(2-Fluor)cyclopropyl-6,7-difluor-1,4-dihydro-4-oxo-3-chinolin-carbonsäure,
1-Cyclopropyl-8-chlor-6,7-difluor-1,4-dihydro-4-oxo-3-chinolin-carbonsäure und
1-Ethyl-6,7,8-trifluor-1,4-dihydro-4-oxo-3-chinolin-carbonsäure
herstellt.

5. Verfahren nach Ansprüchen 1 bis 4, **dadurch gekennzeichnet, dass** man bezogen auf 1 Mol Fluorchinoloncarbonsäure-C₁-C₄-alkylester 20 bis 250 ml Wasser, 200 bis 2000 ml Essigsäure und 2 bis 25 g Schwefelsäure einsetzt, wobei sich die Essigsäuremenge auf 100 %ige Essigsäure und die Schwefelsäuremenge auf 100 %ige Schwefelsäure bezieht.

6. Verfahren nach Ansprüchen 1 bis 5, **dadurch gekennzeichnet, dass** man die Essigsäure in Form von Eisessig und die Schwefelsäure in Form von 96 bis 100 %iger Schwefelsäure einsetzt.

7. Verfahren nach Ansprüchen 1 bis 6, **dadurch gekennzeichnet, dass** man die Destillation so lange betreibt, bis sich bei Normaldruck eine Sumpftemperatur im Bereich von 107 bis 113°C ergibt.

8. Verfahren nach Ansprüchen 1 bis 7, **dadurch gekennzeichnet, dass** man zur Isolierung der hergestellten Fluorchinoloncarbonsäure durch Zugabe einer Base einen pH-Wert im Bereich 2 bis 5 einstellt.

9. Verfahren nach Anspruch 8, **dadurch gekennzeichnet, dass** man zur pH-Wert-Einstellung eine 1 bis 20 gew.-%ige wässrige Natriumacetatlösung verwendet.

10. Verfahren nach Ansprüchen 8 und 9, **dadurch gekennzeichnet, dass** man nach der Einstellung des pH-Wertes auf 0 bis 35°C abkühlt, den dann vorliegenden Niederschlag abfiltriert, mit Wasser wäscht und trocknet.

## Claims

1. Process for the preparation of fluoroquinolonecarboxylic acids by hydrolysis of the corresponding C₁-C₄-alkyl esters with addition of water, acetic acid and sulphuric acid, **characterized in that**, relative to 1 mol of C₁-C₄-alkyl fluoroquinolonecarboxylate, less than 30 g of sulphuric acid are employed, the reaction mixture is heated to reflux for 0.5 to 8 hours, then a mixture of acetic acid, C₁-C₄-alkyl acetate, C₁-C₄-alkyl alcohol and optionally water is distilled off and finally the fluoroquinolonecarboxylic acid prepared is isolated.

2. Process according to Claim 1, **characterized in that** C₁-C₄-alkyl fluoroquinolonecarboxylates of the formula 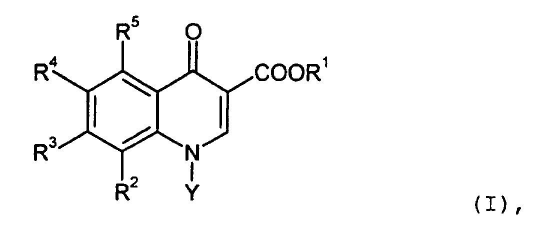 in which
R¹ represents C₁-C₄-alkyl,
R² represents hydrogen, C₁-C₄-alkyl, C₁-C₄-alkoxy, halogen, nitro or cyano,
R³ and R⁴ each represent halogen,
R⁵ represents hydrogen, C₁-C₄-alkyl, halogen or nitro, and
Y represents C₁-C₆-alkyl, cyclopropyl or phenyl, each of which can optionally be substituted by halogen,
where R² and Y together can also represent a -CH₂-CH₂-O- or -CH(CH₃)-CH₂-O- bridge bonded to the nitrogen atom by a C atom and
where at least one of the radicals R² to R⁵ represents fluorine,
are employed and fluoroquinolonecarboxylic acids of the formula 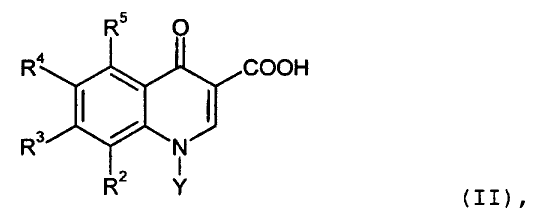 in which the radicals R² to R⁵ and Y have the meaning indicated in formula (I), are prepared.

3. Process according to Claim 2, **characterized in that** in the formulae (I) and (II)
R² represents hydrogen, methyl, methoxy, fluorine, chlorine, nitro or cyano,
R³ represents fluorine or chlorine,
R⁴ represents fluorine,
R⁵ represents hydrogen, methyl, fluorine, chlorine or nitro and
Y represents methyl, ethyl, isopropyl, cyclopropyl, fluorocyclopropyl, 4-fluorophenyl or 2,4-difluorophenyl
and in formula (I) R¹ represents methyl or ethyl.

4. Process according to Claims 1 to 3, **characterized in that** 1-cyclopropyl-6,7,8-trifluoro-1,4-dihydro-4-oxo-3-quinolinecarboxylic acid, 1-cyclopropyl-6,7-difluoro-1,4-dihydro-4-oxo-3-quinoline-carboxylic acid, 1-cyclopropyl-6,7-difluoro-8-cyano-1,4-dihydro-4-oxo-3-quinolinecarboxylic acid, 1-(2-fluoro)cyclopropyl-6,7-difluoro-1,4-dihydro-4-oxo-3-quinolinecarboxylic acid, 1-cyclopropyl-8-chloro-6,7-difluoro-1,4-dihydro-4-oxo-3-quinolinecarboxylic acid and 1-ethyl-6,7,8-trifluoro-1,4-dihydro-4-oxo-3-quinolinecarboxylic acid are prepared.

5. Process according to Claims 1 to 4, **characterized in that**, relative to 1 mol of C₁-C₄-alkyl fluoroquinolonecarboxylate, 20 to 250 ml of water, 200 to 2000 ml of acetic acid and 2 to 25 g of sulphuric acid are employed, the amount of acetic acid relating to 100% strength acetic acid and the amount of sulphuric acid to 100% strength sulphuric acid.

6. Process according to Claims 1 to 5, **characterized in that** the acetic acid is employed in the form of glacial acetic acid and the sulphuric acid in the form of 96 to 100% strength sulphuric acid.

7. Process according to Claims 1 to 6, **characterized in that** the distillation is conducted until a bottom temperature in the range from 107 to 113°C results at normal pressure.

8. Process according to Claims 1 to 7, **characterized in that**, for the isolation of the fluoroquinolonecarboxylic acid prepared, a pH in the range 2 to 5 is set by addition of a base.

9. Process according to Claim 8, **characterized in that**, for setting the pH, a 1 to 20% strength by weight aqueous sodium acetate solution is used.

10. Process according to Claims 8 and 9, **characterized in that** after the setting of the pH to 0 to 35°C the mixture is cooled, and the precipitate then present is filtered off, washed with water and dried.

## Revendications

1. Procédé de préparation d'acides fluoroquinolonecarboxyliques par saponification des C₁-C₄-alkylesters correspondants moyennant addition d'eau, d'acide acétique et d'acide sulfurique qui est **caractérisé en ce que** l'on utilise moins de 30 g d'acide sulfurique par rapport à 1 mole de C₁-C₄-alkylester d'acide fluoroquinolonecarboxylique, on chauffe le mélange réactif pendant 0,5 à 8 heures à reflux, puis on sépare par distillation un mélange d'acide acétique, de C₁-C₄-alkylacétate, de C₁-C₄-alkylalcool et, éventuellement, d'eau, et on isole pour terminer l'acide fluoroquinolonecarboxylique préparé.

2. Procédé selon la revendication 1, **caractérisé en ce que** l'on utilise des C₁-C₄-alkylesters d'acide fluoroquinolonecarboxylique de formule (I) 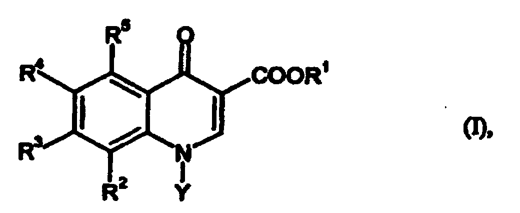 dans laquelle
R¹ désigne un radical C₁-C₄-alkyle,
R² désigne l'hydrogène, un radical C₁-C₄-alkyle, C₁-C₄-alcoxy, un halogène, un radical nitro ou cyano,
R³ et R⁴ désignent respectivement un halogène,
R⁵ désigne l'hydrogène, un radical C₁-C₄-alklyle, un halogène ou un radical nitro et
Y désigne un radical C₁-C₆-alkyle, cyclopropyle ou phényle, qui peut éventuellement être substitué par un halogène,
R² et Y pouvant aussi désigner ensemble un pont -CH₂-CH₂-O- ou -CH(CH₃)-CH₂-O lié avec un atome C à l'atome d'azote et
au moins un des radicaux R² à R⁵ désignant le fluor
et des acides fluoroquinolonecarboxyliques correspondants de formule (II) 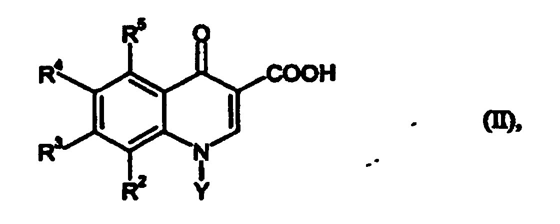 dans laquelle les radicaux R² à R⁵ et Y ont la signification indiquée pour la formule (I).

3. Procédé selon la revendication 2, **caractérisé en ce que**, dans les formules (I) et (II)
R² désigne l'hydrogène, un radical méthyle, méthoxy, le fluor, le chlore ou un radical cyano,
R³ désigne le fluor ou le chlore,
R⁴ désigne le fluor,
R⁵ désigne l'hydrogène, un radical méthyle, le fluor, le chlore ou un radical nitro et
Y désigne un radical méthyle, éthyle, isopropyle, cyclopropyle, fluorocyclopropyle, 4-fluorophényle ou 2,4-difluorophényle,
et R¹ désigne un radical méthyle ou éthyle dans la formule (I).

4. Procédé selon l'une des revendications 1 à 3, **caractérisé en ce que** l'on prépare les acides suivants:
acide 1-cyclopropyl-6,7,8-trifluoro-1,4-dihydro-4-oxo-3-quinoléinecarboxylique,
acide 1-cyclopropyl-6,7-difluoro-1,4-dihydro-4-oxo-3-quinoléinecarboxylique,
acide 1-cyclopropyl-6,7-difluoro-8-cyano-1,4-dihydro-4-oxo-3-quinoléinecarboxylique,
acide 1-(2-fluoro)cyclopropyl-6,7-difluoro-1,4-dihydro-4-oxo-3-quinoléinecarboxylique
acide 1-cyclopropyl-8-chloro-6,7-difluoro-1,4-dihydro-4-oxo-3-quinoléinecarboxylique et
acide 1-éthyl-6,7,8-trifluoro-1,4-dihydro-4-oxo-3-quinoléinecarboxylique.

5. Procédé selon l'une des revendications 1 à 4, **caractérisé en ce que** l'on utilise 20 à 250 ml d'eau, 200 à 2000 ml d'acide acétique et 2 à 25 g d'acide sulfurique pour 1 mole de C₁-C₄-alkylester d'acide fluoroquinolonecarboxylique, la quantité d'acide acétique se rapportant à de l'acide acétique à 100% et la quantité d'acide sulfurique à de l'acide sulfurique à 100%.

6. Procédé selon l'une des revendications 1 à 5, **caractérisé en ce que** l'on utilise l'acide acétique sous la forme d'acide acétique glacial et l'acide sulfurique sous la forme d'acide sulfurique à 96 à 100%.

7. Procédé selon l'une des revendications 1 à 6, **caractérisé en ce que** la distillation est poursuivie jusqu'à ce que l'on obtienne une température de bas de colonne de l'ordre de 107 à 113°C à pression normale.

8. Procédé selon l'une des revendications 1 à 7, **caractérisé en ce que** l'on règle un pH de l'ordre de 2 à 5 par addition d'une base en vue de l'isolation de l'acide fluoroquinolonecarboxylique préparé.

9. Procédé selon la revendication 8, **caractérisé en ce que** l'on utilise une solution aqueuse d'acétate de sodium à 1 à 20% en poids.

10. Procédé selon l'une des revendications 8 et 9, **caractérisé en ce que** l'on refroidit à 0 à 35°C après le réglage du pH, on sépare ensuite le dépôt formé, on le lave à l'eau et on le sèche.
